# FASCICULE DE BREVET EUROPEEN

(11) **EP 1 000 334 B1**
(45) Date de publication et mention de la délivrance du brevet: **30.07.2003**
(21) Numéro de dépôt: 99918035.9
(22) Date de dépôt: 10.05.1999
(51) Int. Cl.: G01N 1/22, C01B 17/04, G01N 33/00

(54) **ANALYSEUR POUR LA MESURE EN CONTINU DE L'H 2?S CONTENU DANS UN GAZ ET DISPOSITIF L'INCLUANT POUR LA REGULATION DU DEBIT D'AIR INJECTE DANS UN REACTEUR D'OXYDATION D'H 2?S EN SOUFRE**
ANALYSEGERÄT ZUR KONTINUIERLICHEN MESSUNG VON H2S IN EINEM GAS UND DESSEN VERWENDUNG IN EINER VORRICHTUNG ZUR REGELUNG DER INJIZIERTEN LUFTMENGE IN EINEN OXYDATIONSREAKTOR ZUR UMSETZUNG VON H2S ZU SCHWEFEL
ANALYZER FOR CONTINUOUSLY MEASURING H 2?S CONTAINED IN A GAS AND DEVICE INCLUDING SAME FOR REGULATING THE AIR FLOW RATE INJECTED INTO AN H 2?S SULPHUR OXIDATION REACTOR

(30) Priorité: 12.05.1998 FR 9805937
(43) Date de publication de la demande: 17.05.2000
(73) Titulaire: Elf Exploration Production, 92400 Courbevoie (FR)
(72) Inventeur: SAVIN-PONCET, Sabine, F-64160 Buros (FR); PEPY, André, F-64230 Lescar (FR); BECOURT, Pierre, F-64320 Bizanos (FR)
(74) Mandataire: Cabinet Hirsch
(86) Numéro de dépôt international: FR9901099
(87) Numéro de publication internationale: WO99058950

(56) Documents cités:
- EP-A- 0 295 173
- EP-A- 0 670 490
- FR-A- 2 118 365
- US-A- 4 101 282
- US-A- 4 402 910
- US-A- 5 473 951
- US-A- 5 637 809

## Description

L'invention a trait à un analyseur pour la mesure en continu de l'H₂S contenu dans un gaz. Elle se rapporte encore à un dispositif incluant ledit analyseur pour la régulation du débit d'air injecté dans un réacteur d'oxydation d'H₂S en soufre.

Pour récupérer l'H₂S présent en faible concentration, notamment concentration inférieure à 5% en volume, dans des gaz de provenances diverses, on fait couramment appel à des procédés mettant en oeuvre une oxydation, notamment oxydation catalytique, de l'H₂S en soufre selon la réaction H₂S + ½ O₂ →S + H₂O.

Dans de tels procédés d'oxydation, on fait passer le gaz à traiter renfermant l'H₂S en présence d'une quantité contrôlée d'un gaz renfermant de l'oxygène libre, au contact d'un catalyseur d'oxydation sélective de l'H₂S en soufre, ledit contact étant réalisé à des températures soit supérieures au point de rosée du soufre formé, auquel cas le soufre formé est présent à l'état de vapeur dans l'effluent gazeux résultant de la réaction, ou bien à des températures inférieures au point de rosée du soufre formé, auquel cas ledit soufre se dépose sur le catalyseur, ce qui nécessite de régénérer périodiquement le catalyseur chargé de soufre par balayage au moyen d'un gaz non oxydant ayant une température comprise entre 200°C et 500°C. Le gaz renfermant de l'oxygène libre utilisé pour l'oxydation de l'H₂S en soufre est le plus souvent de l'air, mais il peut consister également en oxygène, air enrichi en oxygène ou encore mélanges, en proportions variées, d'oxygène et d'un gaz inerte autre que l'azote. Dans ce qui suit, le terme "air" est utilisé pour désigner ledit gaz renfermant de l'oxygène libre.

La quantité d'air, que l'on associe au gaz à traiter renfermant H₂S, est ajustée en continu en réponse à une grandeur résultant de la superposition d'une grandeur de prédiction, représentative d'un débit d'air correspondant à une quantité d'oxygène proportionnelle à la quantité d'H₂S présente dans le gaz à traiter et injectée dans le réacteur d'oxydation, et d'une grandeur de correction (grandeur de contre-réaction), représentative d'un débit d'air correctif pour ramener à une valeur de consigne donnée la teneur de l'H₂S présent dans i'effluent gazeux issu de l'oxydation.

On met en oeuvre l'oxydation dans un réacteur présentant une extrémité amont et une extrémité aval, avantageusement séparées par un lit d'un catalyseur d'oxydation sélective de l'H₂S en soufre, ladite extrémité amont étant équipée d'un premier conduit et d'un second conduit pour l'injection, respectivement, du gaz à traiter et d'air dans le réacteur et ladite extrémité aval étant équipée d'un conduit de sortie pour les gaz pour l'évacuation de l'effluen gazeux résultant de l'oxydation, et l'on assure l'ajustement du débit d'air injecté dans le réacteur d'oxydation à l'aide d'un dispositif de régulation associant (i) un ensemble de prédiction, qui comporte un calculateur de prédiction recevant un signal d'un débitmètre et un signal fourni par un premier analyseur de teneur en H₂S, montés sur le premier conduit à l'extrémité amont du réacteur d'oxydation, et élaborant, à partir desdits signaux, un signal représentatif d'un débit d'air correspondant à une quantité d'oxygène proportionnelle à la teneur en H₂S entrant dans le réacteur d'oxydation, (ii) un ensemble de contre-réaction, qui comporte un calculateur de correction recevant un signal fourni par un second analyseur de teneur en H₂S, monté sur le conduit de sortie du réacteur d'oxydation, et élaborant, à partir dudit signal, un signal représentatif d'un débit d'air correctif pour ramener à une valeur de consigne donnée la teneur de l'H₂S présent dans l'effluent gazeux passant dans ledit conduit de sortie, et (iii) un régulateur de débit recevant les signaux élaborés par les calculateurs de prédiction et de correction et le signal délivré par un débitmètre, monté sur le conduit d'injection d'air à l'extrémité amont du réacteur d'oxydation, ec appliquant à une vanne à ouverture réglable, montée sur ledit conduit d'injection d'air en aval du débitmêtre, un signal de commande de l'ajustement de l'ouverture de ladite vanne, ledit signal de commande étant la résultante des signaux élaborés par les calculateurs de prédiction et de correction.

Les analyseurs, qui sont montés respectivement sur le conduit d'injection du gaz à traiter dans le réacteur d'oxydation et sur le conduit de sortie dudit réacteur, peuvent être, par exemple, des ensembles d'analyse par chromatographie en phase gazeuse (US-A-3026184 et FR-A-2118365), des ensembles d'analyse par spectrométrie différentielle (FR-A-2420754) ou encore des ensembles d'analyse par absorption dans l'infrarouge après transformation sélective de l'H₂S en SO₂.

Les analyseurs des types précités, utilisés pour la mesure de la teneur en H25 dans des gaz renfermant ce composé, ne délivrent pas toujours des signaux continus ou n'offrent pas toujours une sensibilité ou une fiabilité adéquates, de même qu'une simplicité de mise en oeuvre satisfaisante.

Selon l'invention, on propose un analyseur pour la mesure en continu de la teneur en H₂S d'un gaz en renfermant, qui possède une grande sensibilité et dont la réponse ne présente pas de dérive significative dans le temps.

L'analyseur selon l'invention, pour la mesure en continu de la teneur en H₂S d'un échantillon de gaz en renfermant, se caractérise en ce qu'il comporte
- un module de séchage à sec de l'échantillon de gaz comportant une entrée, connectée à une tubulure de prélèvement et d'injection dudit échantillon, et une sortie pour l'échantillon séché,
- un module compresseur présentant un orifice d'aspiration, relié par un conduit à la sortie du module de séchage, et un orifice de refoulement, prolongé par un conduit de circulation de l'échantillon comprimé, ledit conduit étant équipé d'un débicmècre primaire indicateur et/ou régulateur,
- un système de dilution de l'échantillon comprimé comportant un conduit d'amenée d'air, qui est monté en dérivation sur le conduit de circulation de l'échantillon comprimé, en aval du débitmètre primaire , et qui est équipé d'un débitmètre secondaire régulateur ajustant le degré d'ouverture d'une vanne à ouverture réglable montée sur le conduit d'amenée d'air en aval du débitmètre secondaire, et un module régulateur, connecté à chacun des débitmètres primaire et secondaire et asservissant le débitmètre secondaire au débitmètre primaire, et
- un capteur électrochimique de mesure d'H₂S, qui est monté sur le conduit de circulation de l'échantillon comprimé, en aval du conduit d'amenée d'air, et délivre un signal proportionnel à la concentration en H₂S dudit échantillon.

Avantageusement, la tubulure de prélèvement et d'injection de l'échantillon de gaz, connectée à l'entrée du module de séchage à sec, peut être munie, à son extrémité la plus éloignée de ladite entrée, d'un filtre primaire. Eventuellement, un filtre plus fin peut équiper l'autre extrémité de ladite tubulure, située du côté dudit module. Si besoin est ,cette tubulure peut être entourée d'une gaine équipée de moyens de maintien en température par exemple par chauffage électrique ou par circulation d'un fluide caloporteur.

Le module de séchage à sec de l'échantillon de gaz peut consister, en particulier, en un sécheur par membranes de perméation tel que le sécheur "SEC" commercialisé par la société Environnement SA.

Le module compresseur peut être choisi parmi les divers compresseurs miniaturisés ayant les performances requises. Conviennent en particulier les compresseurs à membrane.

Le débitmètre primaire indicateur et/ou régulateur, monté sur le conduit de circulation de l'échantillon comprimé, de même que le débitmètre secondaire régulateur monté sur le conduit d'amenée d'air, sont en particulier des débitmètres massiques. Dans ce cas, le module régulateur, qui leur est associé, est un module régulateur massique.

Le capteur électrochimique de mesure de la concentration d'H₂S est du type transducteur électrochimique de mesure de la pression partielle du composé mesuré. Ce capteur comporte une cellule de mesure, qui contient un électrolyte liquide, dans lequel plongent une électrode de mesure, une électrode de comparaison et une électrode de référence, et qui est séparée, par une membrane, de l'espace de passage de l'échantillon de gaz sur lequel on fait la mesure. Une tension électrique constante est maintenue entre l'électrode de mesure et l'électrode de référence. L'échantillon de gaz renfermant le composé à mesurer, dans le cas présent H₂S, diffuse au travers de la membrane dans l'électrolyte liquide. La tension électrique précitée, l'électrolyte et le matériau des électrodes sont choisis de telle sorte que le composé, dont la concentration est à déterminer, soit transformé électrochimiquement au niveau de l'électrode de mesure et qu'un courant électrique d'intensité proportionnelle à la concentration dudit composé traverse la cellule de mesure. Une réaction électrochimique se produit en même temps, au niveau de l'électrode de comparaison, avec l'oxygène de l'air de dilution. Un tel capteur délivre un signal électrique d'intensité proportionnelle à la concentration du composé à mesurer, ici H₂S, présent dans l'échantillon de gaz passant au contact du capteur. Comme exemple de capteur électrochimique utilisable dans l'analyseur selon l'invention, on peut citer le capteur commercialisé par la société Dràger sous le nom ''Polytron H₂S''.

L'analyseur selon l'invention, qui permet de déterminer la concentration de l'H₂S contenu, en quantité plus ou moins grande, dans un gaz, est tout particulièrement utilisable comme analyseur d'H₂S dans un dispositif de régulation de débit d'air équipant un réacteur d'oxydation d'H₂S en soufre. Plus spécialement, l'analyseur selon l'invention est utilisable pour constituer l'analyseur d'un ensemble de contre-réaction et même l'analyseur d'un ensemble de prédiction du dispositif de régulation ayant la structure définie précédemment, pour réguler le débit d'air injecté dans un réacteur d'oxydation d'H₂S en soufre.

L'invention sera mieux comprise à la lecture de la description donnée ci-après de l'une de ses formes de réalisation, faite en référence aux dessins annexés sur lesquels :
- la figure 1 donne une représentation schématique d'un analyseur selon l'invention, et
- la figure 2 montre schématiquement un réacteur d'oxydation d'H₂S en soufre équipé d'un dispositif de régulation du débit d'air injecté dans le réacteur, ledit dispositif incluant un analyseur tel que représenté sur la figure 1.

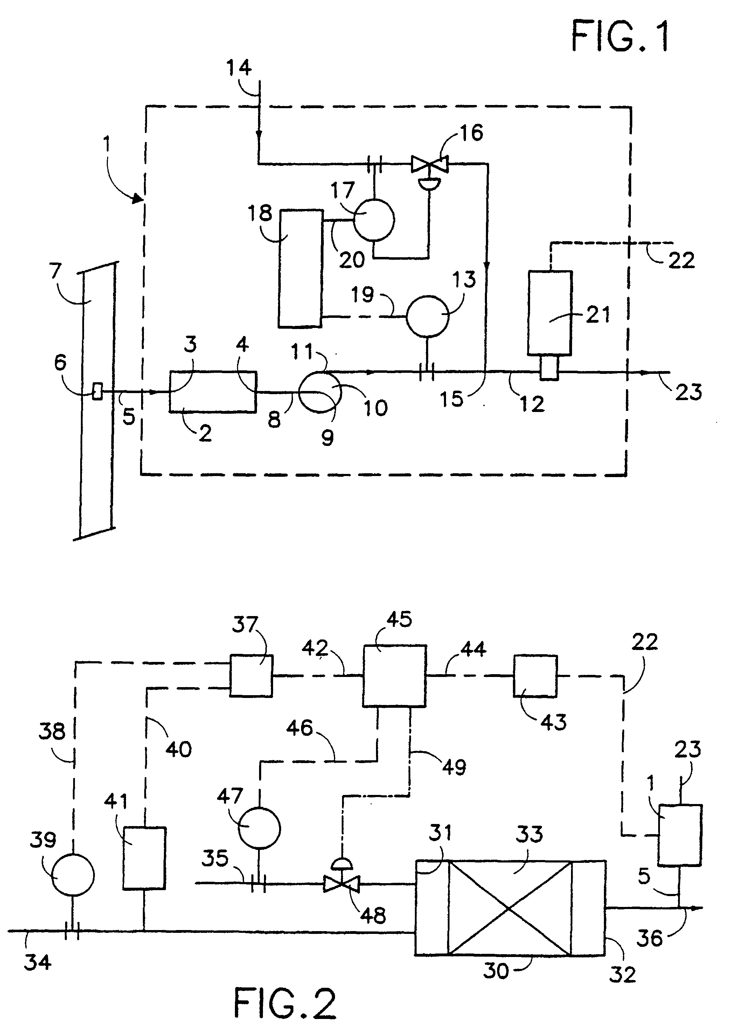

En se référant à la figure 1, l'analyseur 1 représenté comporte un sécheur par membrane de perméation 2 constituant un module de séchage à sec, ledit sécheur présentant une entrée 3 et une sortie 4. L'entrée 3 du sécheur est connectée à une tubulure 5 de prélèvement et d'injection d'échantillon de gaz. L'extrémité de ladite tubulure, qui est la plus éloignée de l'entrée du sécheur, est pourvue d'un filtre primaire 6, à savoir filtre en matériau fritté et notamment en céramique, retenant les particules solides de taille supérieure, par exemple, à 20 µm, et pénètre dans la conduite 7, dans laquelle passe le gaz à échantillonner pour l'analyse. A son extrémité connectée à l'entrée du sécheur 2, la tubulure 5 est munie d'un filtre fin, non représenté, retenant les particules solides de taille supérieure à 0,3 µm, par exemple

La sortie 4 du sécheur 2 est reliée, par un conduit 8, à l'aspiration 9 d'un compresseur à membrane 10 formant module de compression. Ledit compresseur présente un orifice de refoulement 11, qui est prolongé par un conduit 12 de circulation de gaz sur lequel est monté un débitmètre massique primaire 13 indicateur et/ou régulateur. Un conduit 14 d'amenée d'air est monté en dérivation sur le conduit de circulation 12, en un point 15 de ce dernier situé en aval du débitmètre 13, ledit conduit 14 étant muni d'une vanne 16, à degré d'ouverture réglable, et d'un débitmètre massique secondaire régulateur 17 situé en amont de la vanne 16 et assurant l'ajustement du degré d'ouverture de cette dernière. Un module régulateur massique 18 reçoit, par une liaison électrique 19, le signal délivré par le débitmètre primaire 13 et envoie, par une liaison électrique 20, un signal au débitmètre secondaire 17 pour asservir ledit débitmètre secondaire 17 au débitmètre primaire 13. L'ensemble formé du conduit 14 d'amenée d'air avec sa vanne 16 et le débitmètre secondaire 17 et du module régulateur massique 18 constitue un système de dilution pour le contenu du conduit 12.

Un capteur électrochimique 21 de mesure de concentration d'H₂S est monté sur le conduit de circulation 12, en aval du point de jonction 15 dudit conduit avec le conduit 14 d'amenée d'air, ledit capteur délivrant, par un conducteur électrique 22, un signal proportionnel à la concentration en H₂S mesurée.

La partie de la tubulure 5, munie du filtre 6, et la partie 23 du conduit 12 située en aval du capteur21 forment, respectivement, l'entrée de l'analyseur pour l'échantillon gazeux à analyser et la sortie de l'analyseur pour ledit échantillon gazeux, tandis que le conducteur 22 constitue la sortie de mesure de l'analyseur.

En se référant à la figure 2, un réacteur d'oxydation d'H₂S en soufre 30 présente une extrémité amont 31 et une extrémité aval 32 séparées par un lit 33 d'un catalyseur d'oxydation sélective de l'H₂S en soufre, ladite extrémité amont étant équipée d'un premier conduit 34 et d'un second conduit 35 pour l'injection, respectivement, d'un gaz à traiter renfermant H₂S et d'air dans le réacteur et ladite extrémité aval étant équipée d'un conduit 36 de sortie pour les gaz pour l'évacuation de l'effluent gazeux résultant de l'oxydation.

Le réacteur d'oxydation est équipé d'un dispositif de régulation du débit d'air injecté dans le réacteur d'oxydation, ledit dispositif de régulation associant un ensemble de prédiction, un ensemble de contre-réaction et un régulateur de débit d'air.

L'ensemble de prédiction comporte un calculateur de prédiction 37, qui reçoit un signal 38 d'un débitmètre 39 et un signal 40 fourni par un premier analyseur 41 de teneur en H₂S montés sur le premier conduit 34 situé à l'extrémité amont du réacteur d'oxydation 30, et qui élabore, à partir desdits signaux, un signal 42 représentatif d'un débit d'air correspondant à une quantité d'oxygène proportionnelle à la quantité d'H₂S entrant dans le réacteur d'oxydation.

L'ensemble de contre-réaction comporte un calculateur de correction 43, qui reçoit un signal 22 fourni par un second analyseur 1 de teneur en H₂S, monté sur le conduit de sortie 36 du réacteur d'oxydation, et qui élabore, à partir dudit signal 22, un signal 44 représentatif d'un débit d'air correctif pour ramener à une valeur de consigne donnée la teneur de l'H₂S présent dans l'effluent gazeux passant dans ledit conduit de sortie.

Le régulateur de débit 45 reçoit les signaux 42 et 44 élaborés respectivement par les calculateurs de prédiction 37 et de correction 43 et le signal 46 délivré par un débitmètre 47, monté sur le conduit d'injection d'air à l'extrémité amont du réacteur d'oxydation, et applique à une vanne 48 à ouverture réglable, montée sur ledit conduit d'injection d'air en aval du débitmètre 47, un signal de commande 49 de l'ajustement de l'ouverture de ladite vanne, ledit signal de commande étant la résultante des signaux 42 et 44 élaborés respectivement par les calculateurs de prédiction 37 et de correction 43.

L'analyseur 1 de teneur en H₂S, monté sur le conduit de sortie 36 du réacteur d'oxydation, est un analyseur ayant la structure de l'analyseur décrit plus haut en référence à la figure 1. L'analyseur 41 de teneur en H₂S, monté sur le premier conduit 34 situé à l'extrémité amont du réacteur d'oxydation 3C, peut être un analyseur similaire à l'analyseur 1 ou consister en un analyseur d'un autre type, par exemple analyseur par absorption dans l'infrarouge, après transformation sélective de l'H₂S en SO₂, analyseur par chromatographie en phase gazeuse ou encore analyseur par spectrométrie différentielle.

L'analyseur selon l'invention et le dispositif de régulation l'incluant, qui sont décrits ci-dessus, fonctionnent comme indiqué ci-après.

Un échantillon du gaz à analyser renfermant H₂S est prélevé dans le conduit de sortie 36 du réacteur d'oxydation 30, qui correspond à la conduite 7 représentée sur la figure 1, par la tubulure 5, à travers le filtre 6, et ledit échantillon est injecté, par ladite tubulure maintenue par chauffage électrique à une température d'environ 135°C, dans le sécheur 2 par membrane de perméation, dans lequel la vapeur d'eau contenue dans l'échantillon est éliminée quasi complètement. L'échantillon de gaz sec est aspiré et comprimé dans le compresseur à membrane 10 pour être ensuite dirigé dans le conduit de circulation 12, dans lequel, après passage dans le débitmètre massique primaire 13, il est dilué par injection d'air arrivant par le conduit 14 avec un débit massique, qui est asservi, par action du module régulateur 18 agissant sur le débitmètre secondaire régulateur 17 ajustant le degré d'ouverture de la vanne 16, au débit massique de l'échantillon mesuré par le débitmètre 13. Le débit d'air de dilution est choisi de telle sorte que la teneur en H₂S de l'échantillon dilué se trouve dans la plage de concentrations autorisée pour le capteur. L'échantillon gazeux dilué passe ensuite au contact du capteur électrochimique 21 pour la mesure de la concentration en H₂S dudit échantillon, après quoi l'échantillon dilué est dirigé vers une torche, non représentée, pour être brûlé. Le capteur 21 délivre un signal électrique 22 proportionnel à la teneur en H₂S de l'échantillon analysé.

Le calculateur de prédiction 37 reçoit, du débitmètre 39, un signal 38 représentatif du débit de gaz renfermant H₂S injecté dans le réacteur d'oxydation 30 et, de l'analyseur 41, un signal 40 représentatif de la teneur en H₂S dudit gaz et, à partir de ces signaux il élabore un signal 42 représentatif d'un débit d'air correspondant à une quantité d'oxygène proportionnelle à la quantité d'H₂S entrant dans le réacteur d'oxydation. Le coefficient de proportionnalité correspond, en particulier, au rapport molaire O₂:H₂S choisi pour mettre en oeuvre l'oxydation de l'H₂S, ledit rapport pouvant aller, par exemple, de 0,5 à 10 et plus particulièrement de 0,5 à 4. Avantageusement, on peut faire croître progressivement le coefficient de proportionnalité au cours de l'étape d'oxydation, par exemple depuis la valeur 0,5 à la valeur 4, pour éviter une désactivation progressive du catalyseur durant ladite étape.

Le calculateur de correction 43 reçoit, de l'analyseur 1, un signal 22 représentatif de la teneur en H₂S de l'effluent gazeux sortant, par le conduit de sortie 36, du réacteur d'oxydation 30 et, à partir dudit signal, il élabore un signal représentatif d'un débit d'air correctif pour ramener à une valeur de consigne donnée la teneur de l'H₂S présent dans l'effluent gazeux passant dans ledit conduit de sortie.

Le régulateur de débit 45 reçoit les signaux 42 et 44 élaborés respectivement par les calculateurs de prédiction 37 et de correction 43 et le signal 46 délivré par le débitmètre 47, monté sur le conduit 35 d'injection d'air à l'extrémité amont du réacteur d'oxydation 30, et, à partir desdits signaux, il élabore un signal de commande 49, qu'il applique à la vanne 48 à ouverture réglable, montée sur ledit conduit 35 d'injection d'air en aval du débitmètre 47, pour ajuster l'ouverture de ladite vanne, ledit signal de commande 49 étant la résultante des signaux 42 et 44 élaborés respectivement par les calculateurs de prédiction 37 et de correction 43

Dans une variante de mise en oeuvre, le conduit 34 amenant le gaz renfermant H₂S au réacteur d'oxydation, est le conduit de sortie d'un réacteur d'hydrogénation et/ou d'hydrolyse, dans lequel un gaz résiduaire d'usine à soufre est traité pour transformer en H₂S tous les composés soufrés qu'il contient. Dans ce cas, au lieu d'être placé sur ledit conduit 34, le débitmètre 39 peut être monté sur le conduit d'amenée du gaz résiduaire d'usine à soufre au réacteur d'hydrogénation et/ou d'hydrolyse.

## Revendications

1. Analyseur pour la mesure en continu de la teneur en H₂S d'un échantillon de gaz en renfermant **caractérisé en ce qu'**il comporte
- un module de séchage à sec (2) de l'échantillon de gaz comportant une entrée (3), connectée à une tubulure (5) de prélèvement et d'injection dudit échantillon, et une sortie (4) pour l'échantillon séché,
- un module compresseur (10) présentant un orifice d'aspiration (9), relié par un conduit (8) à la sortie du module de séchage, et un orifice de refoulement (11), prolongé par un conduit (12) de circulation de l'échantillon comprimé, ledit conduit étant équipé d'un débitmètre primaire (13) indicateur et/ou régulateur,
- un système de dilution de l'échantillon comprimé comportant un conduit (14) d'amenée d'air, qui est monté en dérivation sur le conduit (12) de circulation de l'échantillon comprimé, en aval du débitmètre primaire, et qui est équipé d'un débitmètre secondaire régulateur (17) ajustant le degré d'ouverture d'une vanne (16) à ouverture réglable montée sur le conduit d'amenée d'air en aval du débitmètre secondaire, et un module régulateur (18), connecté à chacun des débitmètres primaire et secondaire et asservissant le débitmètre secondaire au débitmètre primaire et
- un capteur électrochimique (21) de mesure d'H₂S, qui est monté sur le conduit (12) de circulation de l'échantillon comprimé, en aval du conduit (14) d'amenée d'air, et délivre un signal (22) proportionnel à la concentration en H₂S dudit échantillon.

2. Analyseur selon la revendication 1, **caractérisé en ce que** la tubulure (5) de prélèvement et d'injection de l'échantillon de gaz, connectée à l'entrée du module (2) de séchage à sec, est munie, à son extrémité la plus éloignée de ladite entrée, d'un filtre primaire (6).

3. Analyseur selon la revendication 2, **caractérisé en ce que** l'extrémité de ladite tubulure (5), située du côté du module (2) de séchage à sec, est équipée d'un filtre fin.

4. Analyseur selon l'une des revendications 1 à 3, **caractérisé en ce que** la tubulure (5) de prélèvement et d'injection de l'échantillon de gaz est entourée d'une gaine équipée de moyens de maintien en température, notamment, par chauffage électrique ou par circulation d'un fluide caloporteur.

5. Analyseur selon l'une des revendications l à 4, **caractérisé en ce que** le module (2) de séchage à sec de l'échantillon de gaz consiste en un sécheur par membranes de perméation.

6. Analyseur selon l'une des revendications 1 à 5, **caractérisé en ce que** le module compresseur (10) est un compresseur à membrane.

7. Analyseur selon l'une des revendications 1 à 6, **caractérisé en ce que** le débitmètre primaire (13), monté sur le conduit (12) de circulation de l'échantillon comprimé, de même que le débitmètre secondaire (17), monté sur le conduit (14) d'amenée d'air, sont des débitmètres massiques.

8. Analyseur selon la revendication 7, **caractérisé en ce que** le module régulateur (18), associé au débitmètre primaire (13) et au débitmètre secondaire (17), est un module régulateur massique.

9. Analyseur selon l'une des revendications 1 à 8, **caractérisé en ce qu'**il est intégré, comme analyseur d'H₂S, dans un dispositif de régulation du débit d'air injecté dans un réacteur d'oxydation d'H₂S en soufre

10. Dispositif de régulation du débit d'air injecté dans un réacteur (30) d'oxydation d'H₂S en soufre présentant une extrémité amont (31) et une extrémité aval (32) , ladite extrémité amont étant équipée d'un premier conduit (34) et d'un second conduit (35) pour l'injection, respectivement, du gaz à traiter et d'air dans le réacteur et ladite extrémité aval étant équipée d'un conduit (36) de sortie pour les gaz pour l'évacuation de l'effluent gazeux résultant de l'oxydation, lequel dispositif de régulation est du type comportant (i) un ensemble de prédiction, qui comporte un calculateur de prédiction (37) recevant un signal (38) d'un débitmètre (39) et un signal (40) fourni par un premier analyseur (41) de teneur en H₂S, montés sur le premier conduit (34) à l'extrémité amont du réacteur d'oxydation, et élaborant, à partir desdits signaux, un signal (42) représentatif d'un débit d'air correspondant à une quantité d'oxygène proportionnelle à la teneur en H₂S entrant dans le réacteur (30) d'oxydation, (ii) un ensemble de contre-réaction, qui comporte un calculateur de correction (43) recevant un signal (22) fourni par un second analyseur (1) de teneur en H₂S, monté sur le conduit (36) de sortie du réacteur d'oxydation, et élaborant, à partir dudit signal, un signal (44) représentatif d'un débit d'air correctif pour ramener à une valeur de consigne donnée la teneur de l'H₂S présent dans l'effluent gazeux passant dans ledit conduit de sortie, et (iii) un régulateur de débit (45) recevant les signaux (42, 44) élaborés par les calculateurs de prédiction (37) et de correction (43) et le signal (46) délivré par un débitmètre (47), monté sur le conduit (35) d'injection d'air à l'extrémité amont du réacteur d'oxydation, et appliquant à une vanne (48) à ouverture réglable, montée sur ledit conduit. (35) d'injection d'air en aval du débitmètre (47), un signal (46) de commande de l'ajustement de l'ouverture de ladite vanne, ledit signal de commande étant la résultante des signaux élaborés par les calculateurs de prédiction et de correction, et il se **caractérise en ce qu'**au moins l'analyseur (1) de teneur en H₂S, monté sur le conduit (36) de sortie du réacteur (30) d'oxydation et associé au calculateur de correction (43), est un analyseur selon l'une des revendications 1 à 8.

11. Dispositif de régulation selon la revendication 10, **caractérisé en ce que** le calculateur de prédiction (37) applique un coefficient de proportionnalité entre quantité molaire d'oxygène et quantité molaire d'H₂S allant de 0,5 à 10 et plus particulièrement de 0,5 à 4.

12. Dispositif de régulation selon la revendication 10 ou 11, **caractérisé en ce que** le calculateur de prédiction (37) applique un coefficient de proportionnalité entre quantité molaire d'oxygène et quantité molaire d'H₂S, qui croit progressivement au cours de l'étape d'oxydation.

13. Dispositif de régulation selon l'une des revendications 10 à 12, **caractérisé en ce que** l'analyseur (41) de teneur en H₂S, monté sur le premier conduit (34) à l'extrémité amont du réacteur (30) d'oxydation, est également un analyseur selon l'une des revendications 1 à 8.

14. Dispositif de régulation selon l'une des revendications 10 à 13, **caractérisé en ce que** le conduit (34), amenant le gaz renfermant H₂S au réacteur d'oxydation, est le conduit de sortie d'un réacteur d'hydrogénation et/ou d'hydrolyse, dans lequel un gaz résiduaire d'usine à soufre est traité pour transformer en H₂S tous les composés soufrés qu'il contient et **en ce que** le débitmètre (39), qui est connecté au calculateur de prédiction (37), est monté sur le conduit d'amenée du gaz résiduaire d'usine à soufre au réacteur d'hydrogénation et/ou d'hydrolyse.

## Claims

1. Analyser for continuously measuring the H₂S content of a gas sample containing it, **characterized in that** it comprises:
- a dry-operating module (2) for drying the gas sample, comprising an inlet (3), connected to a nozzle (5) for taking and injecting the said sample, and an outlet (4) for the dried sample;
- a compressor module (10) having a suction port (9), connected via a line (8) to the outlet of the drying module, and a discharge port (11) extended by a flow line (12) for the compressed sample, the said line being equipped with an indicating and/or regulating primary flow meter (13);
- a system for diluting the compressed sample, comprising an air intake line (14), which is mounted as a branch off the flow line (12) for the compressed sample, downstream of the primary flow meter, and which is equipped with a regulating secondary flow meter (17) adjusting the degree of opening of a valve (16) having an adjustable opening, mounted in the air intake line downstream of the secondary flow meter, and a regulating module (18) connected to each of the primary and secondary flow meters and slaving the secondary flow meter to the primary flow meter; and
- an electrochemical sensor (21) for measuring H₂S, which is mounted in the flow line (12) for the compressed sample, downstream of the air intake line (14), and delivers a signal (22) proportional to the concentration of H₂S in the said sample.

2. Analyser according to Claim 1, **characterized in that** the nozzle (5) for taking and injecting the gas sample, connected to the inlet of the dry-operating module (2) for drying the sample, is provided, at its remotest end from the said inlet, with a primary filter (6).

3. Analyser according to Claim 2, **characterized in that** the end of the said nozzle (5), located on the same Side as the dry-operating module (2) for drying the sample, is equipped with a fine filter.

4. Analyser according to one of Claims 1 to 3, **characterized in that** the nozzle (5) for taking and injecting the gas sample is surrounded by a jacket equipped with means for maintaining the temperature, especially by electrical heating or by the circulation of a heat-transfer fluid.

5. Analyser according to one of Claims 1 to 4, **characterized in that** the dry-operating module (2) for drying the gas sample consists of a permeation-membrane dryer.

6. Analyser according to one of Claims 1 to 5, **characterized in that** the compressor module (10) is a diaphragm compressor.

7. Analyser according to one of Claims 1 to 6, **characterized in that** the primary flow meter (13) mounted in the flow line (12) for the compressed sample, as well as the secondary flow meter (17) mounted in the air intake line (14), are mass flow meters.

8. Analyser according to Claim 7, **characterized in that** the regulating module (18), associated with the primary flow meter (13) and with the secondary flow meter (17), is a mass-regulating module.

9. Analyser according to one of Claims 1 to 8, **characterized in that** it is integrated, as an H₂S
analyser, into a device for regulating the flow rate of air injected into a reactor for oxidizing H₂S to sulphur.

10. Device for regulating the flow rate of air injected into a reactor (30) for oxidizing H₂S to sulphur, having an upstream end (31) and a downstream end (32), the said upstream end being equipped with a first line (34) and with a second line (35) for the injection of the gas to be treated and of air into the reactor, respectively, and the said downstream end being equipped with an output line (36) for the gases, in order to discharge the gaseous effluent resulting from the oxidation, which regulating device is of the type having *(i)* a prediction unit, which comprises a prediction computer (37) receiving a signal (38) from a flow meter (39) and a signal (40) delivered by a first H₂S-content analyser these being mounted in the first line (34) at the upstream end of the oxidation reactor, and generating, from the said signals, a signal (42) representative of an air flow rate corresponding to an amount of oxygen proportional to the content of H₂S entering the oxidation reactor (30), (*ii*) a feedback unit, which comprises a correction computer (43) receiving a signal (22) delivered by a second H₂S-content analyser (1), mounted in the output line (36) of the oxidation reactor, and generating, from the said signal, a signal (44) representative of a corrective air flow rate in order to bring the content of the H₂S present in the gaseous effluent flowing through the said output line back to a given set value and *(iii)* a flow regulator (45) receiving the signals (42, 44) generated by the prediction (37) and correction (43) computers and the signal (46) delivered - by a flow meter (47), mounted in the air injection line (35) at the upstream end of the oxidation reactor, and applying to a valve (48) with an adjustable opening, which is mounted in the said air injection line (35) downstream of the flow meter (47), a control signal (46) for adjusting the opening of the said valve, the said control signal being the resultant of the signals generated by the prediction and correction computers, and it is **characterized in that** at least the H₂S-content analyser (1), mounted in the output line (36) of the oxidation reactor (30) and associated with the correction computer (43), is an analyser according to one of Claims 1 to 8.

11. Regulating device according to Claim 10, **characterized in that** the prediction computer (37) applies a coefficient of proportionality between the molar amount of oxygen and the molar amount of H₂S ranging from 0.5 to 10 and more particularly from 0.5 to 4.

12. Regulating device according to Claim 10 or 11, **characterized in that** the prediction computer (37) applies a coefficient of proportionality between the molar amount of oxygen and the molar amount of H₂S, which coefficient gradually increases during the oxidation step.

13. Regulating device according to one of Claims 10 to 12, **characterized in that** the H₂S-content analyser (41), mounted in the first line (34) at the upstream end of the oxidation reactor (30), is also an analyser according to one of Claims 1 to 8.

14. Regulating device according to one of Claims 10 to 13, **characterized in that** the line (34), feeding the H₂S-containing gas into the oxidation reactor, is the output line of a hydrogenation and/or hydrolysis reactor in which a sulphur plant residue gas is treated in order to convert all the sulphur compounds that it contains into H₂S and **in that** the flow meter (39), which is connected to the prediction computer (37) is mounted in the line for feeding the sulphur plant residue gas into the hydrogenation and/or hydrolysis reactor.

## Patentansprüche

1. Analysegerät zur kontinuierlichen Messung des Gehalts an H₂S in einer dieses einschließenden Gasprobe, **dadurch gekennzeichnet, daß** es beinhaltet:
- ein Trocknungsmodul (2) zum Trocknen der Gasprobe, mit einem Einlaß (3), das mit einem Rohr (5) zum Abnehmen und Injizieren der Probe verbunden ist, und mit einem Auslaß (4) für die getrocknete Probe,
- ein Kompressormodul (10), das eine Ansaugöffnung (9), die über eine Leitung (8) mit dem Auslaß des Trocknungsmoduls verbunden ist, und eine Verdrängungsöffnung (11) aufweist, die durch eine Leitung (12) zur Zirkulation der komprimierten Probe verlängert ist, wobei die Leitung mit einem primären Anzeige- und/oder Regulations-Durchflußmesser (13) ausgerüstet ist,
- ein System zur Verdünnung der komprimierten Probe, mit einer Leitung (14) zum Mitführen von Luft, die stromabwärts des primären Durchflußmessers in Form einer Abzweigung über der Leitung (12) zur Zirkulation der komprimierten Probe angebracht ist und die mit einem sekundären Regulations-Durchflußmesser (17) ausgestattet ist, der den Öffnungsgrad eines regulierbaren Öffnungsventils (16), das über der Leitung zum Führen von Luft stromabwärts des sekundären Durchflußmessers angebracht ist, einstellt, und mit einem Regulationsmodul (18), welches sowohl mit dem primären als auch mit dem sekundären Durchflußmesser verbunden ist und den sekundären Durchflußmesser mit dem primären Durchflußmesser regelt, und
- einen elektrochemischen Meßfühler (21) zur Messung von H₂S, der stromabwärts der Leitung (14) zum Führen von Luft über der Leitung (12) zur Zirkulation der komprimierten Probe angebracht ist und ein Signal (22) proportional zur Konzentration von H₂S der Probe liefert.

2. Analysegerät gemäß Anspruch 1, **dadurch gekennzeichnet, daß** das Rohr (5) zum Abnehmen und Injizieren der Gasprobe, welches mit dem Einlaß des Trocknungsmoduls (2) zum Trocknen verbunden ist, an seinem vom besagten Einlaß am weitest entfernten Ende mit einem Primärfilter (6) versehen ist.

3. Analysegerät gemäß Anspruch 2, **dadurch gekennzeichnet, daß** das auf der Seite des Trocknungsmoduls (2) zum Trocknen befindliche Ende des Rohrs (5) mit einem Feinfilter ausgerüstet ist.

4. Analysegerät gemäß einem der Ansprüche 1 bis 3, **dadurch gekennzeichnet, daß** das Rohr (5) zum Abnehmen und zum Injizieren der Gasprobe von einem Mantel umhüllt ist, der mit einem Mittel zum Aufrechterhalten einer Temperatur ausgestattet ist, insbesondere durch eine elektrische Heizung oder durch Zirkulation eines Wärmeträgerfluids.

5. Analysegerät gemäß einem der Ansprüche 1 bis 4, **dadurch gekennzeichnet, daß** das Trocknungsmodul (2) zum Trocknen der Gasprobe sich aus einem Trockner mit Permeationsmembranen zusammensetzt.

6. Analysegerät gemäß einem der Ansprüche 1 bis 5, **dadurch gekennzeichnet, daß** das Kompressormodul (10) ein Membrankompressor ist.

7. Analysegerät gemäß einem der Ansprüche 1 bis 6, **dadurch gekennzeichnet, daß** sowohl der über der Leitung (12) zur Zirkulation der komprimierten Probe angebrachte, primäre Durchflußmesser (13) als auch der über der Leitung (14) zum Führen von Luft angebrachte, zweite Durchflußmesser Massendurchflußmesser sind.

8. Analysegerät gemäß Anspruch 7, **dadurch gekennzeichnet, daß** das Regulationsmodul (18), welches mit dem primären Durchflußmesser (13) und dem sekundären Durchflußmesser (17) verbunden ist, ein Massenregulationsmodul ist.

9. Analysegerät gemäß einem der Ansprüche 1 bis 8, **dadurch gekennzeichnet, daß** es als H₂S-Analysator in eine Vorrichtung zur Regulation der in einen Oxidationsreaktor zur Umsetzung von H₂S in Schwefel injizierten Luftmenge integriert ist.

10. Vorrichtung zur Regulation der in einen Oxidationsreaktor (30) zur Umsetzung von H₂S in Schwefel injizierten Luftmenge, mit einem Stromaufwärtsende (31) und einem Stromabwärtsende (32), wobei das Stromaufwärtsende mit einer ersten Leitung (34) und einer zweiten Leitung (35) zur jeweiligen Injektion von zu behandelndem Gas bzw. Luft in den Reaktor ausgestattet ist und wobei das Stromabwärtsende mit einer Auslaßleitung (36) für die Gase zum Abziehen der aus der Oxidation resultierenden, gasförmigen Ausflüsse ausgestattet ist, wobei die Regulationsvorrichtung so ist, daß sie beinhaltet:
(i) einen Voraussagezusammenbau, der einen Voraussagerechner (37) beinhaltet, welcher ein Signal (38) von einem Durchflußmesser (39) und ein Signal (40), welches durch ein erstes H₂S-Gehalt-Analysegerät (41), das über der ersten Leitung (34) am Stromaufwärtsende des Oxidationsreaktors angebracht ist, geliefert wird, empfängt, und der ausgehend von diesen Signalen ein Signal (42) erarbeitet, welches eine Luftmenge angibt, die einer Sauerstoffmenge entspricht, die proportional zum in den Oxidationsreaktor (30) eintretenden Gehalt an H₂S ist,
(ii) einen Rückkopplungszusammenbau, der einen Korrekturrechner (43) beinhaltet, der ein durch ein zweites, über der Auslaßleitung (36) des Oxidationsreaktors angebrachtes H₂S-Gehalt-Analysegerät (1) geliefertes Signal (22) empfängt und ausgehend von diesem Signal ein Signal (44) erarbeitet, welches repräsentativ ist für eine Korrekturluftmenge, zum Zurückführen eines Einstellwerts, der den Gehalt an H₂S wiedergibt, der im in der Auslaßleitung passierenden Gasausfluß vorliegt, und
(iii) einen Mengenregulator (45), der die Signale (42, 44), die durch den Voraussagerechner (37) und den Korrekturrechner (43) erarbeitet wurden, und das Signal (46), welches durch einen Durchflußmesser (47) geliefert wird, der über der Leitung (35) zur Luftinjektion am Stromaufwärtsende des Oxidationsreaktors angebracht ist, empfängt und auf ein regulierbares Öffnungsventil (48), welches über der Leitung (35) zur Luftinjektion stromabwärts des Durchflußmessers (47) angebracht ist, ein Anweisungssignal (46) zur Einstellung der Öffnung des Ventils anwendet, wobei das Anweisungssignal das Resultat der durch die Voraussage- und Korrekturrechner erarbeiteten Signale ist,
und wobei sie **dadurch gekennzeichnet ist, daß** mindestens
das H₂S-Gehalt-Analysegerät (1), das über der Auslaßleitung (36) des Oxidationsreaktors (30) angebracht ist und mit dem Korrekturrechner (43) verbunden ist, ein Analysegerät gemäß einem der Ansprüche 1 bis 8 ist.

11. Vorrichtung zur Regulation gemäß Anspruch 10, **dadurch gekennzeichnet, daß** der Voraussagerechner (37) einen Proportionalitätskoeffizienten zwischen der molaren Menge an Sauerstoff und der molaren Menge an H₂S anwendet, der im Bereich von 0,5 bis 10 und insbesondere von 0,5 bis 4 liegt.

12. Vorrichtung zur Regulation gemäß Anspruch 10 oder 11, **dadurch gekennzeichnet, daß** der Voraussagerechner (37) einen Proportionalitätskoeffizienten zwischen der molaren Menge an Sauerstoff und der molaren Menge an H₂S anwendet, der im Verlauf der Oxidationsstufe ständig zunimmt.

13. Vorrichtung zur Regulation gemäß einem der Ansprüche 10 bis 12, **dadurch gekennzeichnet, daß** das H₂S-Gehalt-Analysegerät (41), das über der ersten Leitung (34) am Stromaufwärtsende des Oxidationsreaktors (30) angebracht ist, ebenfalls ein Analysegerät gemäß einem der Ansprüche 1 bis 8 ist.

14. Vorrichtung zur Regulation gemäß einem der Ansprüche 10 bis 13, **dadurch gekennzeichnet, daß** die Leitung (34), die das H₂S einschließende Gas in den Oxidationsreaktor mitführt, eine Auslaßleitung eines Hydrierreaktors und/oder Hydrolysereaktors ist, in dem ein Restgas einer Schwefelanlage behandelt wird zum Umwandeln aller Schwefelverbindungen, die sie enthält, in H₂S, und dadurch, daß der Durchflußmesser (39), der mit dem Voraussagerechner (37) verbunden ist, über der Leitung zum Mitführen des Restgases der Schwefelanlage des Hydrier- und/oder Hydrolysereaktors angebracht ist.
